# EUROPEAN PATENT APPLICATION

(11) **EP 0 894 474 A1**
(43) Date of publication of application: **03.02.1999**
(21) Application number: 97112968.9
(22) Date of filing: 28.07.1997
(51) Int. Cl.: A61B 17/00

(54) **Tension holder e.g. for vascular suture or plug device**

(71) Applicant: Ischinger, Thomas, Prof. Dr., 81929 München (DE)
(72) Inventor: Ischinger, Thomas, Prof. Dr., 81929 München (DE)
(74) Representative: Ruschke, Hans Edvard, Dipl.-Ing.

(57) **Abstract**

The invention relates to a device with a mechanism to exert tension upon a string like element such as a suture. The device has its major extension in the plane on which it is positioned and its minor extension along the axis of tension exertion. Said tension mechanism employs lateral or spiral displacement of the string like element, wherein the tensile mechanism has elastic (springy) property, and said mechanism is not substantially extending above the upper surface of the device. This device is particularly used in association with a hemostatic plug to which a string is attached.

## Description

### Background:

With the proliferative use of percutaneous vascular interventions such as Percutaneous Transluminal Coronary Angioplasty (PTCA), angioplasty in other than coronary vessels (PTA), stenting, atherectomy, laser and certain other minimally invasive procedures in cardiology, angiology, cardiosurgery, radiology and surgery as well as other disciplines, a need for percutaneous closure devices of the operative entry site or vascular puncture site has been established.

In particular after percutaneous vascular procedures such as PTCA and PTA a method of nonsurgical closure with or without a suture of the puncture site has proven helpful in reducing bleeding complications, reducing the time necessary to mechanically or manually compress the vascular access site until hemostasis is reached and recucing the time to patient discharge. Vascular closure devices may reduce hosptalization time and may be helpful in performing catheter guided percutaneous procedures on a outpatient basis.

However there are still problems associated with closure devices, which need to be addressed. Closure devices that use sutures applied by needles are not always reliable and may need an additional means of hemostasis such as a collagen or other plug on top of the vascular punture site or in the puncture channel. Such plug, however, carries the risk of getting dislodged and of embolizing into the circulation. This may be a particular risk if repeat procedures have to be carried out through the same punture channel within hours or a few days after the initial procedure. Therefore, closure devices that employ plugs should be associated with a safety mechanism to hold the plug in place. Also, often, it is needed that sutures are being kept under tension for some time to safely achieve hemostasis. Therefore, vascular closure devices featuring needles and sutures as well as vascular closure devices emloying puncture site plugs need a mechansim to hold the suture under tension and the plug safely in place in order to improve efficacy and safety of the closure device. This applies also for closure devices used in other minimally invasive or operative procedures where (percutaneous) closure devices may be of advantage. It is irrelevant for the usefulness of such tension holding device or plug securing device, whether the plug is located totally or partially in the vascular lumen or totally or partially in the puncture site or operative access site channel.
One major feature of such closure device and tension holder and / or plug securing device is, that such device must not prohibit manual compression upon the punture site if needed in case of failure of any of the closure devices. Some of the closure devices are of such a configuration, that no additional manual or any other mechanical compression is possible, since the device is extending significantly above the percutaneous access site (punture site) level. Mechanical compression would critically damage the device and/or inflict trauma to the patient. One solution may be the removal of the part of the device that is extending significantly above the access site level (usually the tension hlding part of the device). However, this carries among other risks e.g.the risk of loosing the plug into the circulation.

It has been found, that the present invention is solving such shortcomings by describing a closure device with a tension hlding part that is not significantly extending above the access site level and that permits application of manual / other mechanical pressure without the risk of reducing the safety and function of the device and without the risk of traumatizing the accesss site and its surrounding tissue (percutaneous punture site area)

### Prior art:

The vascular puncture site sealing device using a collagen plug commercially exploited under the trade name VASOSEAL by Datascope Corp, USA; the combined plug and intravascular achor device, commercially exploited under the trade name ANGIO-SEAL by Sherwood Davies & Geck, USA and the suture devices by Perclose Inc, USA. (including Int application Nr WO 95/13021 and WO 94/13211)
Other prior art includes the device description by Howard Taymor-Luria,USA (US patent 5415657) and by Gene Myers (US patent 5486195); and the international patent applications by David Hathaway et al (Int publication Nr WO 94/08516). These description deal with the use of closure devices and do not describe a suture tension device and/or plug securing device. However, in particular with use of the ANGIO SEAL device, a plug securing and suture tension holding device that conforms to the practical needs is a necessity.

Any description of the present invention will focus on vascular closure devices as they are primarily used after vascular catheterization procedures, yet the description is not limited to such uses; any other use such as after endoscopic procedures, in gyecology, gastroenerology are other operative or minimally invasive procedures may also represent areas of use. "A" always means also more than one.

### Description of invention:

Once a collagen plug is placed within a puncture site channel and associated (attached) with a string (suture) or a plug or anchor is placed intravascularly and is pulled against the puncture site hole from outside via a string or suture or if a suture needs continued tension for a period of time in order to bettr achive hemostasis, a device that exerts continued traction upon such string or suture connection is often needed.

Such tension device will stay in place for minutes, hours or days and will exert the tension while being positioned at the skin level of the patient at or around the percutaneous access site (puncture site). The device needs an atraumatic configuration that minimizes trauma to the tissue it is resting/pushing on. This is particularly important, since by virtue of the tension the device is exerting the device will be pulled against the tissue. Also, the device needs to be flat, so that mechanical pressure (manual pressure) can be exerted (placed) on top of the device. So in the preferred configurations of the device its diameter in the axis of exertion of tension (ie nearly perpendicular to the plane of the surface of the skin) is less than its diameter in the axis of the plane of the skin. In addition to the flat shape, round or oval type configuration help to adapt the device to the configurations of its respective positions, e.g. in the groin. The side of the device facing the skin may be padded for reduced trauma to the tissue and / or lined with a special optimally skin/tissue compatible surface.

Another embodiement is a a open area of the device, i.e. a central or eccentrically located hole or a slit like are that accomodates the string or suture in such a way, that the string or suture can be easily threaded through or into this opening. Once the suture or string (element) upon which the tension is to be exerted is in place, a fixation mechanism is activated in order to both firmly fix the element in its desired distance from the skin (one means to select the degree of tension) and activate the tension mechansim located inside the flat and e.g. olive shaped device.

Commonly the device will be placed just at the skin level with one hand, while the other hand is maintaining slight tension on the string. Once the tension and fixation mechanism is activated, the device is exerting slight tension and both hands can be released.

In one embodiement,the tension exerted by the device - which is dependent on the intial tension of the string that had be held manually and the (elastic) property and the thickness of the tissue between the plug or the suture - may be indicated by a simple gauge on the outside of the device.

The mechanism its self consists of a spring mechanism that displaces the the string from its sraight course to the side (Bajonett ähnlich) or rolls or partially rolls the string up until a tension limit inside the device. The position of the device can be changed; the tension may be determined by positioning (selecting the distance to the skin surface) or by selecting a certain degree of tension with the mechanism itsself prior or after placement of the device.

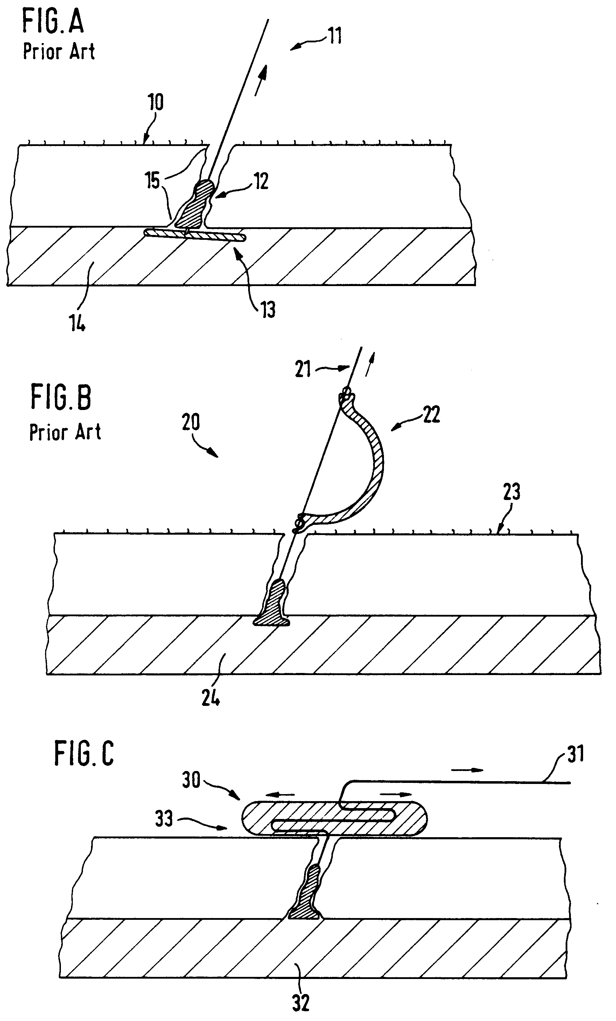

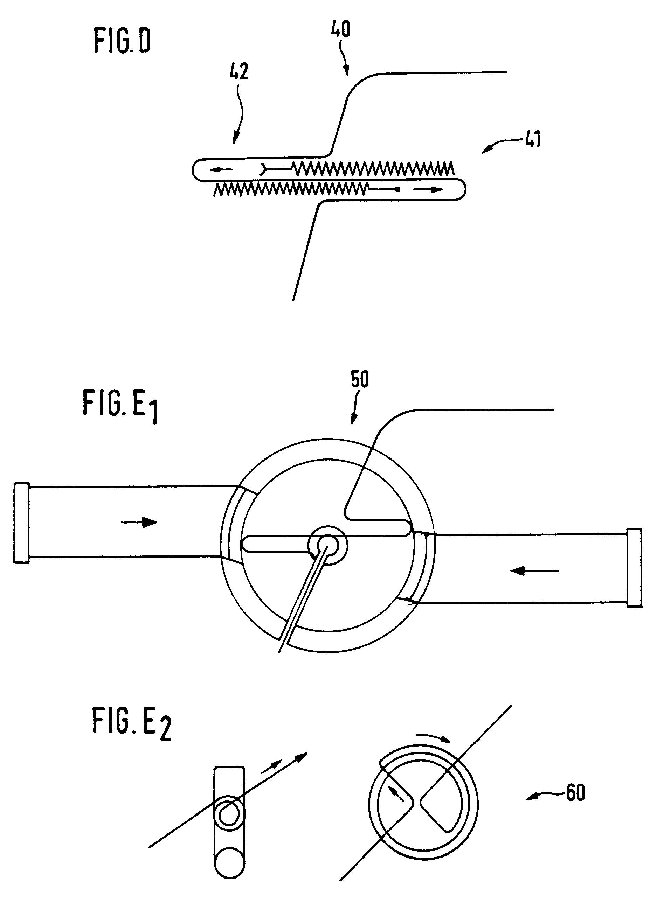

## Claims

1. Device with a mechanism to exert tension upon a string like element such as a suture, wherein the device has its major extension in the plane on which it is positioned and its minor extension along the the axis of tension exertion (blunt, flat configuration), said tension mechanism employing a primarily lateral or spiral displacement of the string like element, wherein the tensile mechanism has elastic (springy) property, and said mechanism is not substantially extending above the upper surface of the device, said tensile mechanism being active upon activation from a non tensile to a tensile (functional) state

2. Device of claim 1 wherein the device is flat oval shaped (flat egg)

3. Device of claim one wherein the device is of a disc like flat shape

4. Device of claim 1 wherein the device permits pressure application on top of it without unacceptable trauma to the underlying tissue

5. Device of claim 1, wherein the side of the device facing the tissue (or in contact with the tissue) is of soft material and/ or tissue compatible material

6. Device of claim 1 used in association with a hemostatic plug to which a string is attached

7. Device of claim 1, wherein the strength of the tensile mechanism can be preselected

8. Device of claim 1 wherein the actual degree of tension is indicated by a gauge

9. Device of claim 1 wherein the tensile mechanism and fixation of the position of the device is activated at the same time

10. Device of claim 1, wherein activation of the tensile mechanism can be done by one hand

11. Device of claim 1, wherein the string to be extended is inserted into the device through a central hole of the device

12. Device of claim 1 wherein the string to be extended is inserted into the device through a slit like opening

13. Device of claim 1 wherein the string to be extended is leaving (exiting from the device) the device at least on one side through a central or nearly central hole or slit like hole
